(19) **Europäisches Patentamt**
**European Patent Office**
**Office européen des brevets**

(11) **EP 4 117 739 B1**

(12) **EUROPEAN PATENT SPECIFICATION**

(45) Date of publication and mention
of the grant of the patent:
**07.01.2026  Bulletin 2026/02**

(51) International Patent Classification (IPC):
**A61L 2/20** *(2006.01)*       **A61L 2/14** *(2006.01)*
**A61L 2/24** *(2006.01)*       **F24F 6/00** *(2006.01)*

(21) Application number: **21712256.3**

(22) Date of filing: **12.03.2021**

(52) Cooperative Patent Classification (CPC):
**A61L 2/20; A61L 2/14; A61L 2/24;** A61L 2202/122;
A61L 2202/123; A61L 2202/14; A61L 2202/15;
A61L 2202/17; A61L 2202/24; F24F 2006/008

(86) International application number:
**PCT/NL2021/050170**

(87) International publication number:
**WO 2021/182960 (16.09.2021 Gazette 2021/37)**

(54) **METHOD AND APPARATUS FOR STERILIZING MEDICAL INSTRUMENTS**

VERFAHREN UND VORRICHTUNG ZUM STERILISIEREN VON MEDIZINISCHEN
INSTRUMENTEN

PROCÉDÉ ET APPAREIL DE STÉRILISATION D'INSTRUMENTS MÉDICAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.03.2020  NL 2025110**

(43) Date of publication of application:
**18.01.2023  Bulletin 2023/03**

(73) Proprietor: **Log10 B.V.**
**5642 JC Eindhoven (NL)**

(72) Inventors:
• **VERHOEVEN, Franciscus Maria**
**5642 JC Eindhoven (NL)**

• **VAN DER LEIJ, Theo Alex Eduard**
**5642 JC Eindhoven (NL)**
• **PEETERS, Mirte**
**5642 JC Eindhoven (NL)**
• **DE JONG, Thijs**
**5642 JC Eindhoven (NL)**

(74) Representative: **V.O.**
**P.O. Box 87930**
**2508 DH Den Haag (NL)**

(56) References cited:
**EP-A1- 3 228 550        EP-A2- 0 387 022
WO-A1-2011/099935     US-A- 5 069 880
US-B1- 8 062 590**

**Description**

FIELD OF THE INVENTION

The present invention relates to sterilizing medical instruments, such as dental instruments. More specifically the invention relates to sterilizing using a plasma source. Even more specifically the invention relates to providing a gas or gas mixture, such as air, having a well-defined specific humidity to the plasma source.

BACKGROUND TO THE INVENTION

Reusable medical instruments are instruments that health care providers can reuse to diagnose and/or treat multiple patients. Examples of reusable medical instruments include medical instruments used in dental care, such as scalpels, syringes, scopes, mirrors, drills, burs, discs, handpieces, excavators, turbines, files, reamers, etc..

When used on patients, reusable instruments become soiled and contaminated with blood, tissue and other biological debris such as microorganisms. To avoid any risk of infection by a contaminated instrument, the reusable instruments can be sterilized. Sterilizing results in a medical instrument that can be safely used more than once in the same patient, or in more than one patient. Adequate sterilizing of reusable medical instruments is vital to protecting patient safety.

Various sterilizing agents can be used for sterilizing medical instruments. Historically, steam or hydrogen peroxide is often used. More recently, plasma devices are being used for ionizing gases or gas mixtures, the ionized gas being used as sterilizing agent. Electrons in the plasma impact on gas molecules causing dissociation and ionization of these molecules, which creates a mix of reactive species. It is known to directly expose the medical instruments to the plasma, or to expose the medical instruments to the (partially) recombined plasma, sometimes referred to as afterglow, see e.g. S. Moreau et al., "Using the flowing afterglow of a plasma to inactivate Bacillus subtilis spores: Influence of the operating conditions", J. Appl. Phys. Vol. 88, No. 2, 15 July 2000.

Several attempts have been made to improve upon plasma sterilizing. US2011/0027125A1 discloses a system comprising a chamber and a plasma generator for generating free radicals combined with use of a hydrogen peroxide solution.

In sterilizing, plasma sources often use a gas or gas mixture, such as air, flowing through the plasma source to be ionized. The moisture content of the gas flow, or specific humidity, can be important in determining the reactive components being generated in the plasma source. However, proper control of the specific humidity of the gas stream tends to be difficult and expensive.

EP3228550 discloses a sterilizing apparatus with a capability for controlling the relative humidity of the fed zone.

There exists a need for more efficient and effective plasma sterilizing.

SUMMARY OF THE INVENTION

It is an object to provide a method and system for sterilizing medical instruments, such as dental instruments. It is an object to provide a more efficient and/or effective method and system for sterilizing medical instruments, such as dental instruments. It is also an object to provide a device and method for controlling specific humidity of a gas stream, particularly for being fed into a plasma source, especially for sterilizing medical instruments.

Thereto, according to an aspect is provided an apparatus for sterilizing medical instruments, such as dental instruments. The sterilizing apparatus includes a device for controlling specific humidity, SH, of flow of a gas or gas mixture, such as air. It has been found that controlling specific humidity of a gas stream fed to an instrument to be sterilized is beneficial for the sterilizing process. Especially when using a plasma sources the specific humidity of the gas flow being ionized by the plasma source can be important in determining the reactive components being generated in the plasma source. The device for controlling specific humidity includes a tank for storing a volume of water. The tank has an inflow port for receiving the gas or gas mixture. The temperature of the water and/or the pressure of the supplied gas, or gas mixture, are adjusted for the required SH. The tank has an outflow port for allowing humidified gas or gas mixture at the required SH to exit the tank.

Specific humidity, SH, or moisture content, is the ratio of the mass of water vapor to the total mass of the humid gas. Specific humidity is a useful parameter, as it relates to the actual amount, i.e. mass, of water vapor present in a given amount of gas. The specific humidity does not vary as the temperature or pressure of a body of air changes, as long as moisture is not added to or taken away from it. Specific humidity, however is difficult to measure. Also it is difficult to set a desired specific humidity. Certain techniques of setting specific humidity rely on in a first step completely removing all moisture from a gas, followed by in a second step evaporating a known amount of moisture into the gas. This can be quite cumbersome and expensive.

Relative humidity, RH, is the ratio of the partial pressure of water vapor to the equilibrium vapor pressure of water at a given temperature. Relative humidity depends on temperature and the pressure of the system of interest. The same amount of water vapor results in higher relative humidity in cool air than warm air. Relative humidity is easier to measure than specific humidity.

The relationship between specific humidity and relative humidity is strongly dependent on temperature and pressure. It has been found that specific humidity can be controlled by controlling temperature, relative humidity and pressure. In particular, when relative humidity is fixed, specific humidity can be controlled by controlling temperature and pressure. Relative humidity, RH, can be fixed at 100% if the gas, or gas mixture, is saturated with water vapor. By providing the tank storing a volume of water and having an inflow port for receiving the gas or gas mixture, the gas or gas mixture can easily be maintained at a relative humidity of 100% inside the tank. The inflow port can be arranged to have the gas or gas mixture enter the volume of water below the water surface. Hence, the having the gas or gas mixture pass through the water aids in having the RH at 100%. The outflow port can be in communication with a gas head inside the tank

The relationship between pressure, temperature and SH for a saturated humid gas or gas mixture is given by the following equation 1.

$$P_{tot}(T, SH) = \frac{A \cdot 10^{\frac{m \cdot T}{T+Tn}}}{1000} \cdot \left(1 + \frac{M_{H2O}}{Mdry} \cdot \left(\frac{1000}{SH} - 1\right)\right) \qquad \text{EQ1}$$

Herein $P_{tot}$ is the total pressure of the humid gas or gas mixture (in bar(a)), T is the temperature of the humid gas or gas mixture (in °C), SH is the specific humidity of the humid gas or gas mixture (in grams of water vapor per kilogram of gas), $M_{dry}$ is the molar mass of the dry gas or gas mixture (in g/mol), and $M_{H2O}$ is the molar mass of water (in g/mol). The parameters A, m and $T_n$ are constants. The below table gives values for these constants for water, for several temperature ranges.

| A | m | $T_n$ | max error | Temperature range |
|---|---|---|---|---|
| 6.116441 | 7.591386 | 240.7263 | 0.083% | -20...+50°C |
| 6.004918 | 7.337936 | 229.3975 | 0.017% | +50...+100°C |
| 5.856548 | 7.27731 | 225.1033 | 0.003% | +100...+150°C |
| 6.002859 | 7.290361 | 227.1704 | 0.007% | +150...+200°C |

In a practical example, e.g. for water at 40°C, $A = 6.116441$, $m = 7.591386$, $T_n = 240.7263$, $M_{dry} = 28.9444$ for air g/mol, and $M_{H2O} = 18.01534$ g/mol. From this relationship the required pressure can be calculated for a desired SH at a given temperature. Similarly, from this relationship the required temperature can be calculated for a desired SH at a given pressure.

As stated, when relative humidity is fixed specific humidity can be controlled by controlling temperature and pressure. Therefore, using this relationship there is no need to first remove all moisture from a gas and subsequently add a known amount of moisture. Instead is relied on the known saturation of the gas with water vapor and its relation with the controllable temperature and/or pressure. It will be appreciated that it is possible that temperature is maintained and pressure is adjusted for the desired SH. It is also possible that pressure is maintained and temperature is adjusted for the desired SH. It is also possible that both temperature and pressure are adjusted for the desired SH.

Optionally, the apparatus includes a processor arranged for receiving a desired SH value as input and for determining suitable values for temperature and/or pressure, e.g. on the basis of equation EQ1. The processor can be arranged for receiving or determining the desired SH value and for determining values for a required temperature and/or required pressure on the basis of the desired SH value, e.g. on the basis of equation EQ1.

Optionally, the apparatus includes a temperature sensor and temperature controller for maintaining the water at the controlled temperature. When the water temperature is maintained at a controlled value, variations in SH can be minimized. The processor can be arranged for receiving a desired SH value and the controlled temperature value as input, and for determining a required pressure, on the basis of the desired SH and the controlled temperature, e.g. on the basis of equation EQ1.

Optionally, the apparatus includes a pressure regulator, e.g. in communication with the inflow port. Hence, the pressure of the gas or gas mixture in the tank can be controlled and maintained. The processor can be arranged controlling the pressure to be the required pressure as determined on the basis of the desired SH and the controlled temperature, e.g. on the basis of equation EQ1. When the gas pressure inside the tank is maintained at a controlled value, variations in SH can be minimized.

It will be appreciated that it is possible that the processor is arranged for receiving a desired SH value the controlled pressure value as input, and for determining a required temperature, on the basis of the desired SH and the controlled pressure, e.g. on the basis of equation EQ1. The processor can then be arranged for controlling the temperature to be the required temperature as determined on the basis of the desired SH and the controlled pressure, e.g. on the basis of equation EQ1.

Optionally, the apparatus includes a water supply port for maintaining the water level within a predetermined interval. Preferably, the water supply port includes a level meter for determining the water level in the tank. Thus the water level can automatically be maintained.

Optionally the apparatus includes a plasma source having an ionization chamber in communication with the device for controlling specific humidity. Optionally, the outflow port of the device is connected to a gas input of the ionization chamber via a choke. Thus, the gas or gas mixture having the controlled SH can be fed to the ionization chamber of the plasma source. The controlled SH can help in setting or maintaining the reactive components being generated in the plasma source.

It will be appreciated that the processor can be arranged for receiving an indication representative of desired sterilization parameter and determining a desired SH value on the basis thereof. Such sterilization parameter can e.g. be a desired reactive component content of the plasma. The reactive component content of the plasma can be dependent on the SH of the gas or gas mixture fed to the ionization chamber of the plasma source. The processor can e.g. look up a desired SH value corresponding to the desired sterilization parameter, such as the desired reactive component content, in a database or memory. It will be appreciated that the indication representative of the desired sterilization parameter can e.g. be one of a set of predetermined settings of the sterilization apparatus. The predetermined settings can e.g. relate to different instruments to be sterilized, different grades of contamination of the instruments to be sterilized, different levels of sterilization, or the like.

Optionally, the outflow port of the device is connected to a gas input of the ionization chamber via a choke. Thus, pressure inside the tank can be maintained while the gas flow can be fed to the ionization chamber at a reduced pressure relative to the pressure inside the tank.

Optionally, the apparatus includes one or more of a RH sensor, temperature sensor, pressure sensor, and flow sensor in a connection from the outflow port of the device to a gas input of the ionization chamber. These sensors can be used for verifying the RH, temperature, pressure, and/or flow of gas input to the ionization chamber.

Optionally, the apparatus includes a chamber arranged for placing the medical instrument therein. The apparatus can include a temperature control unit arranged for controlling the temperature of the medical instrument and/or the chamber such that the temperature of the medical instrument is below the temperature of chamber for allowing the sterilizing agent to at least partially condense onto the instrument.

Optionally, the apparatus further includes a washing unit arranged for washing and/or rinsing the medical instruments prior to sterilization. A cooling gas stream, e.g. including atomized water, can be supplied to the washed medical instruments for drying and cooling the medical instruments.

It will be appreciated that the device for controlling specific humidity of flow of a gas or gas mixture, including a tank for storing a volume of water, wherein the tank has an inflow port for receiving the gas or gas mixture, wherein the temperature of the water and/or the pressure of the supplied gas or gas mixture are controlled for the required SH, and wherein the tank has an outflow port for allowing humidified gas or gas mixture at the required SH to exit the tank, can also be used for supplying a flow of gas having controlled SH in different applications than in a sterilization apparatus for sterilizing medical instruments. Such device can e.g. be used in combination with a plasma source. It will be appreciated that a plasma source can be provided having an ionization chamber, one or more electrodes, and an electric power source. The ionization chamber can have an input for feeding humidified gas, such as humidified air, into the ionization chamber. The plasma source can include a humidifier providing the humidified gas to the ionization chamber. The humidifier includes a tank for storing a volume of water, wherein the tank has an inflow port for receiving a gas stream, and an outflow port for allowing humidified gas at a required SH to exit the tank. The temperature of the water and/or the pressure of the supplied gas are controlled for the required SH.

According to an aspect is provided a method for sterilizing a medical instrument. The method includes placing the medical instrument in a chamber and supplying a humidified gas stream to the medical instrument. The humidified gas stream has a controlled specific humidity. The gas stream having the controlled specific humidity is obtained by providing a gas stream into a tank storing a volume of water, controlling a temperature of the water and/or a pressure of the supplied gas for the required specific humidity, and flowing the humidified gas stream having the controlled specific humidity from the tank.

More in general is provided a method for controlling specific humidity of a gas stream. The method includes providing a gas stream into a tank storing a volume of water. The method includes controlling a temperature of the water and/or a pressure of the supplied gas for the required specific humidity. The method includes flowing the humidified gas stream having the controlled specific humidity from the tank.

It will be appreciated that any of the aspects, features and options described in view of the apparatus apply equally to the device and method, and vice versa. It will also be clear that any one or more of the above aspects, features and options can be combined.

BRIEF DESCRIPTION OF THE DRAWINGS

Embodiments of the present invention will now be described in detail with reference to the accompanying drawings in which:

Figure 1 shows a schematic representation of an example of an apparatus for sterilizing a medical instrument;
Figure 2 shows a schematic representation of an example of a humidifier;
Figure 3 shows a schematic representation of a method;
Figure 4 shows a schematic representation of a method;
Figure 5 shows an example of pressure curves;
Figure 6 shows a schematic representation of an example of an apparatus for sterilizing a medical instrument; and
Figure 7 shows a schematic representation of an example of an apparatus for sterilizing a medical instrument.

DETAILED DESCRIPTION

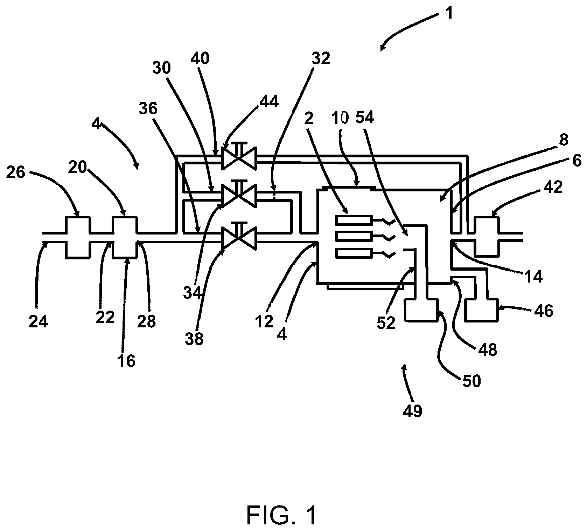

Figure 1 shows an example of a schematic view of a sterilization apparatus 1 for sterilizing medical instruments, such as dental instruments 2. The apparatus 1 includes a sterilizing chamber 4. The sterilizing chamber 4 is arranged for placing the medical instrument 2 to be sterilized therein. In this example, the chamber 4 is arranged for placing a plurality of medical instruments 2 to be sterilized therein. Here, the medical instruments 2 are dental instruments. The chamber 4 includes walls 6 forming an internal space 8 for receiving the medical instruments 2. In this example, the chamber 4 has a door 10 for allowing the medical instruments 2 to be inserted into and extracted from the internal space 8 of the chamber 4. The chamber 4 includes a sterilizing agent supply port 12. The chamber 4 includes an exhaust port 14.

The apparatus 1 includes a sterilizing agent source 16. The sterilizing agent source 16 is arranged for providing a sterilizing agent. In this example, the sterilizing agent includes at least partially recombined ionized humidified air. In this example, the sterilizing agent source 16 includes a plasma source 20. The plasma source 20 includes an output port 28 in communication with the sterilizing agent supply port 12 of the chamber 4.

A first duct 30 extends between the plasma source 20 output port 28 and the sterilizing agent supply port 12 of the chamber 4. The first duct 30 includes a choke 32. In this example, the first duct 30 further includes a first valve 34. The first valve 34 is arranged for selectively opening and closing the first duct 30. Here the first valve 34 is positioned upstream of the choke 32. It will be clear that alternatively the first valve 34 may be positioned downstream of the choke 32.

The choke 32 is arranged for causing choked flow of the sterilizing agent from the plasma source 20 to the chamber 4 when the chamber is at the reduced pressure. The choked flow is a fluid dynamic condition associated with the Venturi effect. When a gas stream at a given pressure and temperature passes through a choke, also referred to as restriction or constriction, into a lower pressure environment the gas velocity increases. At initially subsonic upstream conditions, the conservation of mass principle requires the gas velocity to increase as it flows through the smaller cross-sectional area of the choke. At the same time, the Venturi effect causes the static pressure, and therefore the density, to decrease at the choke. Choked flow is a limiting condition where the mass flow will not increase with a further decrease in the downstream pressure for a fixed upstream pressure and temperature. The physical point at which the choking occurs for adiabatic conditions is when the velocity at the exit of the choke is at sonic conditions; i.e., at a Mach number of 1. Hence, thanks to the choke 32 the pressure in the plasma source 20 does not decrease below a predetermined threshold pressure when the pressure in the chamber 4 is reduced further below such predetermined threshold pressure.

Steady-state choked flow occurs when the pressure downstream of the choke falls below a critical value relative to the pressure upstream of the choke. The critical pressure value p* can be calculated from the following equation.

$$\frac{p^*}{p_0} = \left(\frac{2}{\gamma+1}\right)^{\frac{\gamma}{\gamma-1}} \qquad\qquad EQ2$$

Herein $p_0$ is the absolute upstream pressure and y the heat capacity ratio $c_p/c_v$ of the gas or gas mixture. For air the heat capacity ratio y is about 1.4. Thus for air p* = $0.528p_0$.

When the gas velocity is choked, the equation for the mass flow rate is as follows.

$$\dot{m} = C_d A \sqrt{\gamma \rho_0 p_0 \left(\frac{2}{\gamma+1}\right)^{\frac{\gamma+1}{\gamma-1}}} \qquad\qquad EQ3$$

Herein m is the mass flow rate in kg/m2, $C_d$ the discharge coefficient (dimensionless), A the cross-sectional area of the hole in m², $\rho_0$ the real gas density at total pressure $p_0$ and temperature $T_0$ in kg/m³, and $T_0$ the absolute upstream temperature of the gas in K. The discharge coefficient $C_d$ is the ratio of the actual discharge to the theoretical discharge, i.e. the ratio of the mass flow rate at the discharge end of the nozzle to that of an ideal nozzle which expands an identical working fluid from the same initial conditions to the same exit pressures. The discharge coefficient $C_d$ is generally in the range of 0.5-1. The Discharge coefficient can e.g. be on the order of 0.6 (e.g. sharp edged orifice) to 0.8 (e.g. longer hole). The discharge coefficient for a specific choke can easily be determined by comparing a measured mass flow rate to the theoretical mass flow rate ($C_d$ = 1).

Hence, by properly designing the flow restriction properties of the choke 32 it is possible to prevent the pressure in the plasma source 20 to decrease below the threshold pressure, while the sterilizing chamber 4 is evacuated. Thus, the plasma source 20 can be operated continuously at nearly constant pressure, e.g. at near-ambient pressure. Hence, composition of the at least partially recombined gas mixture from the plasma source 20 can remain constant, or at least nearly constant, i.e. sufficiently constant, despite reducing the pressure in the sterilizing chamber 4.

In the example of Figure 1 a second duct 36 extends between the plasma output port 28 and the sterilizing agent supply port 12 of the chamber 4. The second duct 36 includes a second valve 38. The second valve 38 is arranged for selectively opening and closing the second duct 36.

In the example of Figure 1 a third duct 40 extends between the plasma output port 28 and a destructor 42. The destructor 42 is arranged for destructing airborne components. In this example, the third duct 40 further includes a third valve 44. The third valve 44 is arranged for selectively opening and closing the third duct 40. In Figure 1 the destructor 42 is further connected to the exhaust port 14 of the chamber 4 for destructing any contaminants carried by the exhausted sterilizing agent.

In this example, a pump 46 is connected to a pumping port 48 of the chamber 4. The apparatus 1 in this example includes a temperature control unit 49. In this example, the temperature control unit 49 includes a cooling unit 50. The cooling unit 50 is arranged for cooling the medical instruments 2. In Figure 1, the cooling unit 50 is arranged for cooling the instruments 2 prior to placing the instruments 2 in the chamber 4. The cooling unit 50 in this example includes a gas conduit 52 for cooling the medical instrument using a gas, here air. The gas conduit 52 includes a mouth 54, here nozzles, pointing a stream of the gas onto the medical instrument 2.

The plasma source 20 includes an input port 22 for feeding a humidified air stream into the plasma source 20. In Fig. 1 the input port 22 is connected to an air stream supply 24 via a humidifier 26. In this example, the humidifier 26 is a device for controlling specific humidity, SH, of a flow of a gas therethrough. It will be appreciated that the gas can be a gas mixture. It will be appreciated that the gas, or gas mixture, can include a vapor.

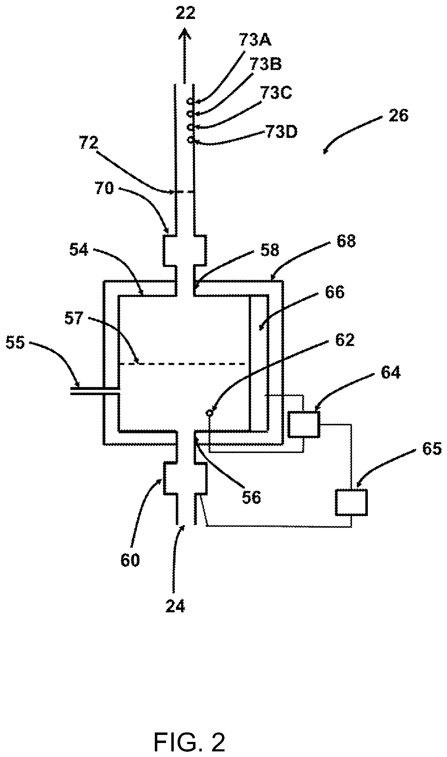

Figure 2 shows a schematic depiction of a more detailed example of a humidifier. The humidifier 26 includes a tank 54. In this example the tank 54 is partially filled with water. The tank has an inflow port 56 for receiving the gas, here from the air stream supply 24. The air stream supply 24 can e.g. be a compressed air supply, e.g. at approximately 6 bar(a). Here the inflow port 56 is arranged to have the gas enter the volume of water below the water surface 57. The tank 54 has an outflow port 58 for allowing humidified gas at the required SH to exit the tank 54. Here, the outflow port 58 is in communication with a gas head inside the tank 54. Here, the apparatus includes a water supply port 55 for maintaining the water level within a predetermined interval. The water supply port 55 can be provided with an automatic filling device, e.g. a float operated valve or a liquid level operated valve for automatically maintaining the water level with a predetermined interval.

By flowing, e.g. bubbling, the gas through the water, the relative humidity, RH, of the gas will be at, or near, 100%. Also, by flowing the gas through the water, the temperature of the gas will be equal to, or close to, the temperature of the water. It will be appreciated that a person of skill in the art can easily determine, e.g. on the basis of some simple experiments, a volume of liquid required for ensuring that in the gas head of the tank the relative humidity, RH, of the gas will be at 100%, and that the temperature of the gas will be equal to the temperature of the water.

In the example of Fig. 2 the humidifier 26 includes a pressure regulator 60 in communication with the inflow port 56. Here the pressure regulator 60 is a control valve that reduces the input pressure of a gas to a desired value at its output. Here the pressure regulator 60 is adjustable, such that a pressure at the output of the regulator can be adjusted to a desired value. Thus the pressure in the tank 54 can be adjusted to a desired value.

In the example of Fig. 2 the humidifier 26 is arranged for controlling the temperature of the water in the tank 54. Here, the humidifier 26 includes a temperature sensor 62, a temperature controller 64, and a heater 66. A thermal insulation 68 may be provided around the tank 54 for conserving energy and for minimizing temperature fluctuations inside the tank 54. The temperature controller 64 is arranged for controlling heat of the heater 66 for maintaining the water at a controlled temperature on the basis of a temperature measured by the temperature sensor 62. Here the temperature at which the controller 64 maintains the water is adjustable, such that the temperature of the water can be adjusted to a desired value. Thus the temperature of the water in the tank 54 can be adjusted to a desired value.

The specific humidity, SH, of the gas in the gas head in the tank 54 is dependent on the relative humidity, RH, of the gas, the pressure and the temperature. As described above, the RH can be set to 100% in the tank. The temperature of the gas can be set by setting the temperature of the water in the tank, e.g. using the temperature controller 64. The pressure of the gas in the gas head can be set by setting the feed pressure using the pressure regulator 60. For example, for a desired specific humidity of 10 grams of water vapor per kilogram of gas, and a water temperature of 40 °C, using equation EQ1 the required pressure can be calculated to be $P_{tot}(40, 10) = 4.623$ bar(a). Thus, by setting the pressure regulator 60 to 4.623 bar(a) and setting the temperature controller 64 to 40 °C, gas flow having a specific humidity of 10 g/kg can be obtained.

In the example of Fig. 2 the humidifier 26 further includes a processor 65. The processor 65 is arranged for receiving or determining a desired SH value and for determining values for a required temperature and/or required pressure on the basis of the desired SH value. The processor can e.g. determine a combination of suitable required temperature and required pressure on the basis of equation EQ1 as set out hereinabove. The processor can be arranged to simultaneously control both the temperature and the pressure and maintain both at the required value. It is also possible that the processor monitors the pressure, in real-time (or at least with minimum delay time) determines the required temperature for obtaining the desired SH at the measured pressure, and control the temperature to be at the required temperature in real-time. It is also possible that the processor monitors the temperature, in real-time (or at least with minimum delay time) determines the required pressure for obtaining the desired SH at the measured temperature, and control the pressure to be at the required pressure in real-time.

In this example, the processor is arranged for receiving an indication representative of a desired sterilization parameter. The desired sterilization parameter can e.g. be a desired reactive component content of the plasma. The indication representative of the desired sterilization parameter can e.g. be one of a set of predetermined settings of the sterilization apparatus. The predetermined settings can e.g. relate to different instruments to be sterilized, different grades of contamination of the instruments to be sterilized, different levels of sterilization, or the like. The processor in this example determines the desired SH value on the basis of the indication representative of the desired sterilization parameter. The processor can e.g. look up the desired SH value corresponding to the desired sterilization parameter in a database or memory.In the example of Fig. 2 a further pressure regulator 70 is provided at the outflow port 58 of the humidifier 26. Here, the further pressure regulator is set to a pressure that is lower than the pressure inside the tank 54. Thus, the humidified gas can be expelled from the humidifier 26 at a regulated pressure that is independent from the gas pressure inside the tank. Hence, feed pressure towards the plasma source and pressure selected for setting the desired SH can be chosen, and controlled, independently. Further, in the example of Fig. 2 a second choke 72 is in communication between the outflow port 58 of the humidifier 26 and the input port 22 of the plasma source 20. This second choke 72 can be used for further reducing the pressure of the humidified gas fed towards the plasma source 20. Further, the second choke 72 can be dimensioned to define a mass flow of humidified gas to the plasma source.

It is noted that when reducing the pressure and/or temperature of the gas stream, such as downstream of the tank 54, the SH of the gas stream does not change. The RH of the gas stream, however, does change by said reducing of the pressure and/or temperature. Therefore, piping connecting the tank to the plasma source may be thermally insulated for preventing saturation, or even condensation, of the gas stream to occur upstream of, or in, the plasma source 20.

In this example, a connection from the outflow port 58 of the humidifier 26 to the input port 22 of the plasma source 20 includes one or more of a RH sensor 73A, a temperature sensor 73B, a pressure sensor 73C, and a flow sensor 73D. These sensors can be used for verifying a mass flow towards the plasma source and/or for verifying the SH of the gas flowing to the plasma source 20. The SH can be calculated from the measured RH, temperature and pressure, on the basis of equation EQ1.

In this example, the SH value determined from the readings of the sensors 73A, 73B, 73C is used for verification only. In this example, there is no data connection between the sensors 73A, 73B, 73C and the processor 65 or controller 64. It will be appreciated that it is also possible to use the determined SH value for adjusting the pressure and or temperature in the tank 54 for controlling the SH.

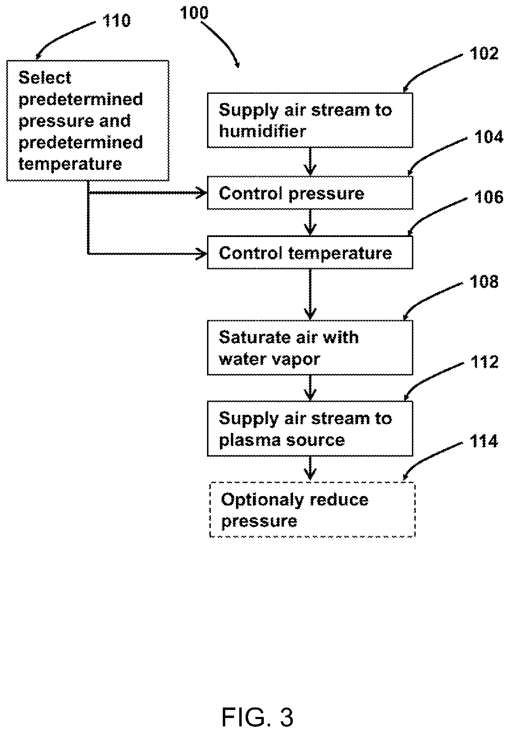

The apparatus as described in relation to Figures 1 and 2 can be used in the following exemplary first method 100, see Figure 3. An air stream is supplied 102 from the air stream supply 24 via the pressure regulator 60 to the inflow port 56 of the humidifier 26 for setting the specific humidity of the air stream to a predetermined SH value. The pressure of the air stream is controlled 104 by the pressure regulator 56 to a predetermined pressure value. The air stream enters the tank 54 below water level. The water is controlled to be at a predetermined temperature value by temperature controller 64. Thereby the temperature of the air in the tank 54 is controlled 106 to the predetermined temperature value. Also, the air is saturated 108 with water vapor, i.e. RH becomes 100% at the predetermined pressure and predetermined temperature. Depending on the humidity of the air supplied to the humidifier 26, the humidifier 26 can add or remove water from the air such that at the exit of the humidifier 26 an air stream with a predetermined SH is obtained. The predetermined pressure and predetermined temperature had been chosen 110 such that, at saturation, the specific humidity of the gas corresponds to the predetermined SH value.

The gas having the predetermined SH value is fed 112 from the outflow port 58 of the humidifier 26, towards the plasma source 20. Here, the pressure of the gas stream is reduced 114 by the further pressure regulator 70 and the second choke 72. The air stream entering the plasma source 20 has the predetermined SH value. The specific humidity of the air entering the plasma source 20 can e.g. be 10 $\pm$ 1 g/kg (grams of water per kg of air).

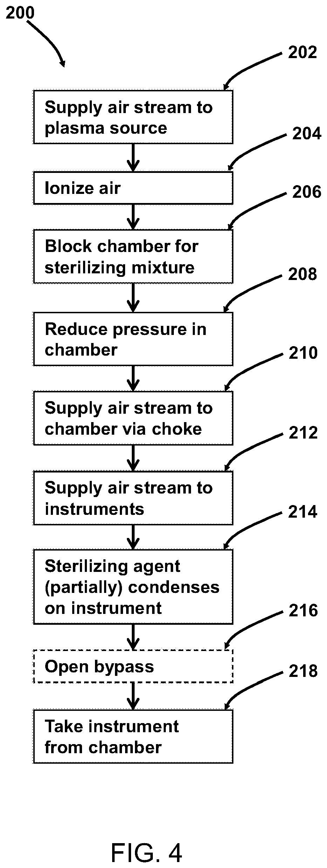

According to a second method 200, see Figure 4, the gas having the predetermined SH value is fed 202 from the outflow port 58 of the humidifier 26, to the plasma source 20. In the plasma source 20 the air is at least partially ionized 204. The ionized air is fed to the output port 28. With the first and second valves 34, 38 closed, preventing 206 the sterilizing mixture to enter the chamber 4, the sterilizing mixture may be fed to the destructor 42 via the third duct 40 with the third valve 44 opened. Hence, the plasma source 20 can be operated continuously. The sterilizing mixture can be fed to the chamber 4 when needed, and fed to the destructor 42 when not needed in the chamber 4. Thus, the plasma source 20 is immediately ready for providing the sterilizing mixture 18 to the chamber 4 when required.

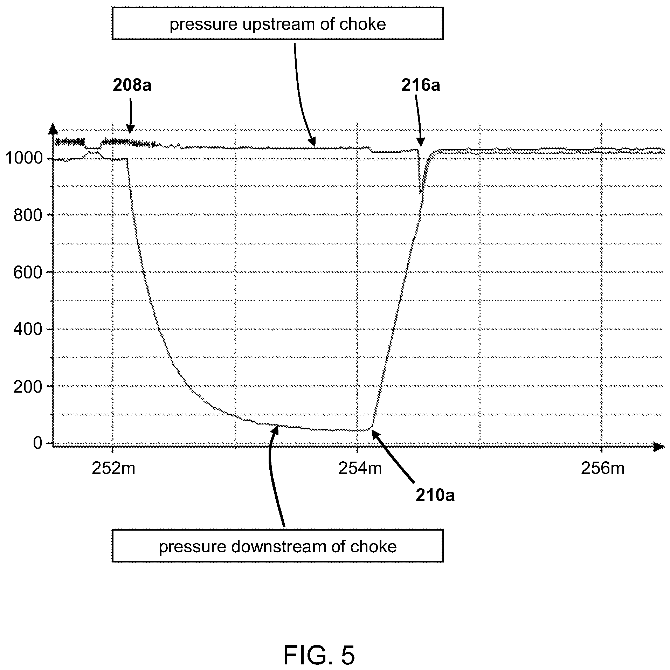

The medical instruments 2 to be sterilized are placed inside the chamber 4. Then, the pressure in the chamber 4 is reduced 208 by the pump 36. In this example the pressure is reduced to approximately 50 mbar Figure 5 shows a graph of the pressure as a function of time upstream of the choke 32, here the pressure in the plasma source 20, (upper curve) and the pressure downstream of the choke 32, here in the sterilization chamber 4 (lower curve). Figure 5 shows that the pressure in the chamber 4 starts to decrease from the moment 208a the pump is started. It is noted that the pressure upstream of the choke 32 does not decrease yet as the first and second valves 30, 36 are still closed in this example. This removes a large portion of the gasses that entered the chamber 4 while placing the medical instruments 2 therein. Next, the first valve 34 is opened at moment 210a. Hence, the sterilizing mixture is fed 210 into the chamber 4 via the choke 32. Thus, the pressure in the chamber 4 will be raised by feeding the sterilizing mixture into the chamber 4 as can be seen in Figure 4. The choke 32 in this example is dimensioned such that the pressure upstream of the choke 32 is maintained at approximately 1 bar. Hence, the pressure in the plasma source 20 can in this example not drop below a predetermined threshold pressure of approximately 1 bar. Here the flow through the choke is limited to about 10 liters per minute at 1 bar. Thereto, in this example, the choke has a round aperture of 0.785 mm.

In this example, with the first valve opened, the flow of the ionized humidified air from the plasma source 20, at least partly, recombines while flowing into the chamber 4. The sterilizing agent formed by the at least partly recombined ionized humidified air then contacts 212 the medical instruments 2 to be sterilized. Optionally, if the medical instruments 2 are, or have been, cooled, the sterilizing agent, at least partially, condenses 214 onto the medical instruments 2 and sterilizes the medical instruments 2. If the walls 6 of the chamber 4 are not cooled, less cooled than the medical instruments, or even heated, condensation of the sterilizing agent onto the walls 6 can be prevented.

In this example, as an option, the second valve 38 is opened 216 at moment 216a when the pressure in the chamber 4 rises to about 850 mbar, i.e. when the pressure in the chamber exceeds a predetermined threshold opening pressure of, here, 850 mbar. The opening of the second valve causes sterilizing agent to rush into the chamber 4 via the second duct 36, i.e. bypassing the choke 32. When the second valve 38 is open, the first valve 34 may be closed if desired. Hence, when the pressure in the chamber 4 is not too low, i.e. here not below the predetermined threshold opening pressure of in this example 850 mbar, the second valve 38 can be opened to allow a greater mass flow of sterilizing mixture than is achievable through the choke 32. As the pressure in the chamber 34 is above the threshold opening pressure at that time, the pressure in the plasma source cannot drop below the threshold opening pressure.

The medical instruments 2 may be taken from the chamber 218 immediately or may remain in the chamber 4 for some time for additional exposure to the sterilizing agent. The sterilizing agent may be fed to the destructor 42 via the exhaust port 14.

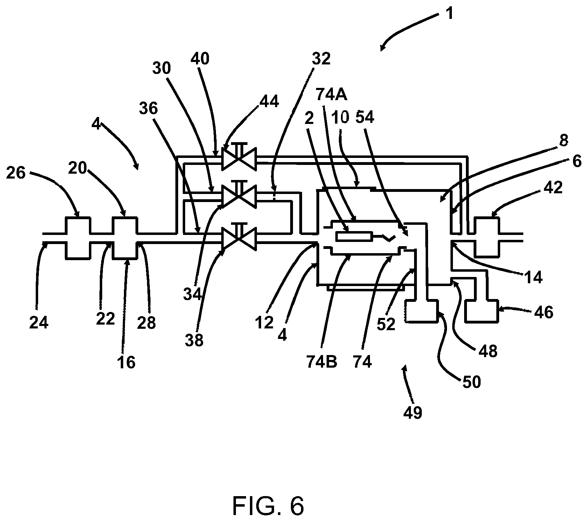

Figure 6 shows a schematic representation of an example of an apparatus 1 for sterilizing a medical instrument 2. The example of Figure 6 is similar to the example of Figure 1. A main difference is that the apparatus 1 of Figure 6 further includes a container 74. The container 74 is arranged for holding the medical instrument 2, here for holding a plurality of medical instruments 2. The container in this example includes a tray 74A and a lid 74B. The container 74 can be opened by removing the lid 74B from the tray 74A for placing one or more medical instruments 2 inside the container 74. The container 74 is arranged for being placed in the chamber 4. The chamber 4 can include guides for holding the container 74. The apparatus 1 in this example is arranged for opening the container inside the chamber 4. In the example of Figure 6 the cooling unit 10 is arranged for cooling the container 74 to below the temperature of the chamber 4. Hence, the medical instruments 2 in the container 74 can easily be cooled together with the container 74. Also, hence the container 74, which can be contaminated as well, can easily be sterilized.

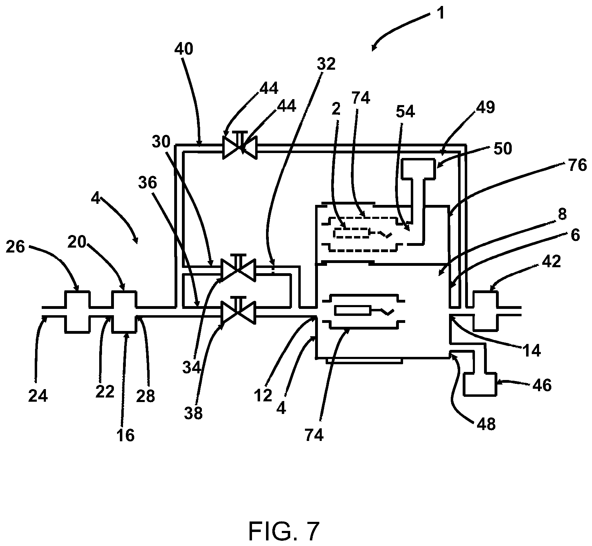

Figure 7 shows a schematic representation of an example of an apparatus 1 for sterilizing a medical instrument 2. The example of Figure 7 is similar to the example of Figure 6. A main difference is that the apparatus 1 of Figure 7 further includes a cooling chamber 76. The cooling chamber 76 is arranged for holding the medical instrument 2, here for holding the container 74 holding medical instruments 2 while cooling the medical instrument(s) 2 and optionally the container 74. In this example the medical instruments 2, here in the container 74, are cooled in the cooling chamber 58 and then transferred to the chamber 4 for sterilization. The apparatus 1 can include a handler unit for transferring the medical instruments 2 and/or the container 74 from the cooling chamber 76 to the sterilization chamber 4 after cooling.

Herein, the invention is described with reference to specific examples of embodiments of the invention. It will, however, be evident that various modifications and changes may be made therein, without departing from the essence of the invention. For the purpose of clarity and a concise description features are described herein as part of the same or separate embodiments, however, alternative embodiments having combinations of all or some of the features described in these separate embodiments are also envisaged.

In the example of Figure 1 the gas stream having the predetermined SH value is fed from the humidifier to the plasma source. It is also possible that the gas stream having the predetermined SH value is fed from the humidifier to the chamber without passing a plasma source.

It will be appreciated that the humidifier as described herein can also be used in alternative sterilizing apparatus than described in view of Figures 1, 6 and 7.

In the example of Figure 1 the cooling unit is arranged for cooling the medical instrument in the chamber. It is also possible that alternatively, or additionally, the cooling unit is arranged for cooling the medical instrument prior to placing the medical instrument inside the chamber, e.g. as disclosed in view of Figure 7.

It is possible that the apparatus further includes a washing unit arranged for washing and/or rinsing the medical instruments prior to sterilization. Preferably, the medical instruments are dried prior to sterilization. The cooling gas stream, optionally including the atomized water, can be supplied to the washed medical instruments for drying and cooling the medical instruments.

However, other modifications, variations, and alternatives are also possible. The specifications, drawings and examples are, accordingly, to be regarded in an illustrative sense rather than in a restrictive sense.

In the claims, any reference signs placed between parentheses shall not be construed as limiting the claim. The word 'comprising' does not exclude the presence of other features or steps than those listed in a claim. Furthermore, the words 'a' and 'an' shall not be construed as limited to 'only one', but instead are used to mean 'at least one', and do not exclude a plurality.

## Claims

1. A sterilization apparatus for sterilizing medical instruments, including:

   a device for controlling specific humidity, SH, of flow of a gas or gas mixture, including a tank for storing a volume of water,
   wherein the tank has an inflow port for receiving the gas or gas mixture;
   wherein the temperature of the water and/or the pressure of the supplied gas or gas mixture are controlled for the required SH; and
   wherein the tank has an outflow port for allowing humidified gas or gas mixture at the required SH to exit the tank.

2. The apparatus of claim 1, wherein the inflow port is arranged to have the gas or gas mixture enter the volume of water below the water surface, and/or wherein the outflow port is in communication with a gas head inside the tank.

3. The apparatus of claim 1 or 2, including a processor arranged for retrieving a desired SH value and for determining values for a required temperature and/or required pressure on the basis of the desired SH value.

4. The apparatus of any of claims 1-3, including a temperature sensor, and temperature controller for maintaining the water at a controlled temperature.

5. The apparatus of claims 3 and 4, wherein the processor is arranged for receiving the controlled temperature value as input, and for determining the required pressure on the basis of the desired SH and the controlled temperature.

6. The apparatus of any of claims 1-5, including a pressure regulator for maintaining the pressure in the tank at a controlled pressure.

7. The apparatus of claims 3 and 6, wherein the processor is arranged for receiving the controlled pressure value as input, and for determining the required temperature on the basis of the desired SH and the controlled pressure.

8. The apparatus of any of claims 3-7, wherein the processor is arranged for controlling the temperature and/or pressure in the tank on the basis of the required temperature and/or required pressure.

9. The apparatus of any of claims 3-8, wherein the processor is arranged for receiving an indication representative of desired sterilization parameter and determining the desired SH value on the basis thereof.

10. The apparatus of any of claims 1-9, including a water supply port for maintaining the water level within a predetermined interval.

11. The apparatus of any of claims 1-10, including a plasma source having an ionization chamber in communication with the device for controlling specific humidity.

12. The apparatus of claim 11, wherein (i) the outflow port of the device is connected to a gas input of the ionization chamber via a choke, and/or (ii) including one or more of a RH sensor, temperature sensor, pressure sensor, and flow sensor in a connection from the outflow port of the device to a gas input of the ionization chamber.

13. The apparatus according to any of claims 1-12, including:

   - a chamber arranged for placing the medical instrument therein; and
   - a temperature control unit arranged for controlling the temperature of the medical instrument and/or the chamber such that the temperature of the medical instrument is below the temperature of chamber for allowing the sterilizing agent to at least partially condense onto the instrument.

14. A method for sterilizing a medical instrument, including:

   - placing the medical instrument in a chamber;
   - supplying a humidified gas stream to the medical instrument, wherein the humidified gas stream has a controlled specific humidity and is obtained by:

      - providing a gas stream into a tank storing a volume of water,
      - controlling a temperature of the water and/or a pressure of the supplied gas for the required specific humidity,
      - flowing the humidified gas stream having the controlled specific humidity from the tank.

15. The method of claim 14, including having the gas stream enter the tank below the water surface, and/or including flowing the humidified gas stream having the controlled specific humidity from a gas head inside the tank.

16. The method of claim 14 or 15, including using a processor for retrieving a desired SH value and for determining values for a required temperature and/or required pressure on the basis of the desired SH value.

17. The method of any of claims 14-16, using a temperature sensor, and temperature controller for maintaining the water at the controlled temperature.

18. The method of claims 16 and 17, including the processor receiving the controlled temperature value as input, and determining the required pressure on the basis of the desired SH and the controlled temperature.

19. The method of any of claims 14-17, including controlling the gas pressure using a pressure regulator for maintaining the pressure in the tank at a controlled pressure.

20. The method of claims 16 and 19, including the processor receiving the controlled pressure value as input, and determining the required temperature on the basis of the desired SH and the controlled pressure.

21. The method of any of claims 14-20, including (i) the processor controlling the temperature and/or pressure in the tank on the basis of the required temperature and/or required pressure, and/or (ii) the processor receiving an indication representative of desired sterilization parameter and determining the desired SH value on the basis thereof, and/or (iii) at least partly ionizing the humidified gas stream having the controlled specific humidity using a plasma source prior to supplying a humidified gas stream to the medical instrument.

22. The method of claim 21, flowing the humidified gas stream having the controlled specific humidity from the tank to the plasma source via a choke.

23. The method of any of claims 14-22, including automatically maintaining the water level in the tank within a predetermined interval.

24. The method of claim 21 or 22, including measuring one or more of relative humidity, temperature, pressure, and flow in a connection from the tank to the plasma source.

**25.** The method according to any of claims 14-24, including controlling a temperature of the medical instrument and/or the chamber such that the temperature of the medical instrument is below the temperature of chamber for allowing the sterilizing agent to at least partially condense onto the instrument.

**Patentansprüche**

**1.** Vorrichtung zum Sterilisieren von medizinischen Instrumenten, umfassend:

Vorrichtung zur Regelung der spezifischen Feuchte SH eines Gas- oder Gasgemischstroms, einschließlich eines Tanks zur Speicherung einer Wassermenge,
wobei der Tank eine Einlassöffnung zum Aufnehmen des Gases oder Gasgemisches aufweist;
wobei die Temperatur des Wassers und/oder der Druck des zugeführten Gases oder Gasgemisches für die erforderliche SH geregelt werden; und
wobei der Tank eine Auslassöffnung aufweist, um befeuchtetes Gas oder das Gasgemisch mit der erforderlichen SH aus dem Tank austreten zu lassen.

**2.** Vorrichtung nach Anspruch 1, wobei die Einlassöffnung so angeordnet ist, dass das Gas oder Gasgemisch unterhalb der Wasseroberfläche in das Wasservolumen eintritt, und/oder wobei die Auslassöffnung mit einem Gasraum innerhalb des Tanks in Verbindung steht.

**3.** Vorrichtung nach Anspruch 1 oder 2, einschließlich eines Prozessors, der zum Abrufen eines gewünschten SH-Werts und zum Bestimmen von Werten für eine erforderliche Temperatur und/oder einen erforderlichen Druck auf der Grundlage des gewünschten SH-Werts ausgelegt ist.

**4.** Vorrichtung nach einem der Ansprüche 1 bis 3, mit einem Temperatursensor und einem Temperaturregler, um das Wasser auf der geregelten Temperatur zu halten.

**5.** Vorrichtung nach den Ansprüchen 3 und 4, wobei der Prozessor so ausgelegt ist, dass er den geregelten Temperaturwert als Eingabe empfängt und den erforderlichen Druck auf der Grundlage des gewünschten SH-Werts und der geregelten Temperatur bestimmt.

**6.** Vorrichtung nach einem der Ansprüche 1 bis 5, einschließlich eines Druckreglers zum Aufrechterhalten des Drucks im Tank auf einem geregelten Druck.

**7.** Vorrichtung nach den Ansprüchen 3 und 6, wobei der Prozessor so ausgelegt ist, dass er den geregelten Druckwert als Eingabe empfängt und die erforderliche Temperatur auf der Grundlage des gewünschten SH-Werts und des geregelten Drucks bestimmt.

**8.** Vorrichtung nach einem der Ansprüche 3 bis 7, wobei der Prozessor so ausgelegt ist, dass er die Temperatur und/oder den Druck im Tank auf der Grundlage der erforderlichen Temperatur und/oder des erforderlichen Drucks regelt.

**9.** Vorrichtung nach einem der Ansprüche 3 bis 8, wobei der Prozessor so ausgelegt ist, dass er eine Angabe empfängt, die für den gewünschten Sterilisationsparameter repräsentativ ist, und auf dieser Grundlage den gewünschten SH-Wert bestimmt.

**10.** Vorrichtung nach einem der Ansprüche 1 bis 9, mit einer Wasserversorgungsöffnung zum Aufrechterhalten des Wasserstands innerhalb eines vorbestimmten Intervalls.

**11.** Vorrichtung nach einem der Ansprüche 1 bis 10, einschließlich einer Plasmaquelle mit einer Ionisationskammer, die mit der Vorrichtung zum Steuern der spezifischen Feuchtigkeit in Verbindung steht.

**12.** Vorrichtung nach Anspruch 11, wobei (i) die Auslassöffnung der Vorrichtung über eine Drossel mit einem Gaseinlass der Ionisationskammer verbunden ist und/oder (ii) ein oder mehrere der folgenden Elemente enthalten sind: ein RH-Sensor, ein Temperatursensor, ein Drucksensor und ein Durchflusssensor in einer Verbindung von der Auslassöffnung der Vorrichtung zu einem Gaseinlass der Ionisationskammer.

**13.** Vorrichtung nach einem der Ansprüche 1 bis 12, umfassend:

- eine Kammer, die zum Platzieren des medizinischen Instruments darin angeordnet ist; und
- eine Temperatursteuereinheit, die zum Steuern der Temperatur des medizinischen Instruments und/oder der Kammer angeordnet ist, so dass die Temperatur des medizinischen Instruments unter der Temperatur der Kammer liegt, damit das Sterilisationsmittel zumindest teilweise auf dem Instrument kondensieren kann.

14. Verfahren zum Sterilisieren eines medizinischen Instruments, umfassend: - Platzieren des medizinischen Instruments in einer Kammer;

- Zuführen eines befeuchteten Gasstroms zu dem medizinischen Instrument, wobei der befeuchtete Gasstrom eine geregelte spezifische Feuchtigkeit aufweist und erhalten wird durch:

- Bereitstellen eines Gasstroms in einen Tank, der eine Wassermenge speichert
- Regeln einer Temperatur des Wassers und/oder eines Drucks des zugeführten Gases für die erforderliche spezifische Feuchtigkeit
- Strömenlassen des befeuchteten Gasstroms mit der geregelten spezifischen Feuchtigkeit aus dem Tank.

15. Verfahren nach Anspruch 14, wobei der Gasstrom unterhalb der Wasseroberfläche in den Tank eintritt und/oder der befeuchtete Gasstrom mit der geregelten spezifischen Feuchtigkeit aus einem Gaskopf innerhalb des Tanks strömt.

16. Verfahren nach Anspruch 14 oder 15, wobei ein Prozessor verwendet wird, um einen gewünschten SH-Wert abzurufen und Werte für eine erforderliche Temperatur und/oder einen erforderlichen Druck auf der Grundlage des gewünschten SH-Werts zu bestimmen.

17. Verfahren nach einem der Ansprüche 14 bis 16, wobei ein Temperatursensor und ein Temperaturregler verwendet werden, um das Wasser auf der geregelten Temperatur zu halten.

18. Verfahren nach den Ansprüchen 16 und 17, wobei der Prozessor den geregelten Temperaturwert als Eingabe empfängt und den erforderlichen Druck auf der Grundlage des gewünschten SH-Werts und der geregelten Temperatur bestimmt.

19. Verfahren nach einem der Ansprüche 14 bis 17, wobei der Gasdruck unter Verwendung eines Druckreglers geregelt wird, um den Druck im Tank auf einem geregelten Druck zu halten.

20. Verfahren nach den Ansprüchen 16 und 19, wobei der Prozessor den geregelten Druckwert als Eingabe empfängt und die erforderliche Temperatur auf der Grundlage des gewünschten SH-Werts und des geregelten Drucks bestimmt.

21. Verfahren nach einem der Ansprüche 14 bis 20, umfassend (i) den Prozessor, der die Temperatur und/oder den Druck im Tank auf der Grundlage der erforderlichen Temperatur und/oder des erforderlichen Drucks steuert, und/oder (ii) den Prozessor, der eine Angabe empfängt, die für den gewünschten Sterilisationsparameter repräsentativ ist, und auf dieser Grundlage den gewünschten SH-Wert bestimmt, und/oder (iii) das zumindest teilweise Ionisieren des befeuchteten Gasstroms mit der geregelten spezifischen Feuchtigkeit unter Verwendung einer Plasmaquelle, bevor ein befeuchteter Gasstrom dem medizinischen Instrument zugeführt wird.

22. Verfahren nach Anspruch 21, wobei der befeuchtete Gasstrom mit der geregelten spezifischen Feuchtigkeit über eine Drossel vom Tank zur Plasmaquelle fließt.

23. Verfahren nach einem der Ansprüche 14 bis 22, einschließlich der automatischen Aufrechterhaltung des Wasserstands im Tank innerhalb eines vorbestimmten Intervalls.

24. Verfahren nach Anspruch 21 oder 22, einschließlich der Messung einer oder mehrerer der folgenden Größen: relative Feuchtigkeit, Temperatur, Druck und Durchfluss in einer Verbindung vom Tank zur Plasmaquelle.

25. Verfahren nach einem der Ansprüche 14 bis 24, einschließlich der Regelung einer Temperatur des medizinischen Instruments und/oder der Kammer, so dass die Temperatur des medizinischen Instruments unter der Temperatur der Kammer liegt, damit das Sterilisationsmittel zumindest teilweise auf dem Instrument kondensieren kann.

**Revendications**

1. Appareil de stérilisation destiné à stériliser des instruments médicaux, comportant :

   un dispositif destiné à contrôler l'humidité spécifique, SH, d'un flux de gaz ou de mélange gazeux, comportant un réservoir destiné à stocker un volume d'eau,
   dans lequel le réservoir possède un orifice d'admission destiné à recevoir le gaz ou le mélange gazeux ;
   dans lequel la température de l'eau et/ou la pression du gaz ou du mélange gazeux fourni sont contrôlées en termes d'humidité spécifique requise ; et
   dans lequel le réservoir possède un orifice d'évacuation destiné à permettre au gaz ou au mélange gazeux humidifié à l'humidité spécifique requise de sortir du réservoir.

2. Appareil selon la revendication 1, dans lequel l'orifice d'admission est prévu de sorte que le gaz ou le mélange gazeux pénètre dans le volume d'eau sous la surface de l'eau, et/ou dans lequel l'orifice d'évacuation est en communication avec une tête de gaz à l'intérieur du réservoir.

3. Appareil selon la revendication 1 ou 2, comportant un processeur prévu pour rechercher une valeur d'humidité spécifique souhaitée et pour déterminer des valeurs pour une température requise et/ou une pression requise sur la base de la valeur d'humidité spécifique souhaitée.

4. Appareil selon l'une quelconque des revendications 1 à 3, comportant un capteur de température, et un contrôleur de température destiné à maintenir l'eau à une température contrôlée.

5. Appareil selon les revendications 3 et 4, dans lequel le processeur est prévu pour recevoir la valeur de température contrôlée en guise d'entrée, et pour déterminer la pression requise sur la base de l'humidité spécifique souhaitée et de la température contrôlée.

6. Appareil selon l'une quelconque des revendications 1 à 5, comportant un régulateur de pression destiné à maintenir la pression dans le réservoir à une pression contrôlée.

7. Appareil selon les revendications 3 et 6, dans lequel le processeur est prévu pour recevoir la valeur de pression contrôlée en guise d'entrée, et pour déterminer la température requise sur la base de l'humidité spécifique souhaitée et de la pression contrôlée.

8. Appareil selon l'une quelconque des revendications 3 à 7, dans lequel le processeur est prévu pour contrôler la température et/ou la pression dans le réservoir sur la base de la température requise et/ou de la pression requise.

9. Appareil selon l'une quelconque des revendications 3 à 8, dans lequel le processeur est prévu pour recevoir une indication représentative d'un paramètre de stérilisation souhaité et déterminer la valeur d'humidité spécifique souhaitée sur la base de celui-ci.

10. Appareil selon l'une quelconque des revendications 1 à 9, comportant un orifice d'alimentation en eau destiné à maintenir le niveau d'eau dans un intervalle prédéterminé.

11. Appareil selon l'une quelconque des revendications 1 à 10, comportant une source de plasma ayant une chambre d'ionisation en communication avec le dispositif destiné à contrôler l'humidité spécifique.

12. Appareil selon la revendication 11, dans lequel (i) l'orifice d'évacuation du dispositif est relié à une entrée de gaz de la chambre d'ionisation via un étrangleur, et/ou (ii) comportant un ou plusieurs d'un capteur d'humidité relative, d'un capteur de température, d'un capteur de pression et d'un capteur de débit reliés entre l'orifice d'évacuation du dispositif et une entrée de gaz de la chambre d'ionisation.

13. Appareil selon l'une quelconque des revendications 1 à 12, comportant :

   - une chambre prévue pour placer l'instrument médical à l'intérieur ; et
   - une unité de contrôle de la température prévue pour contrôler la température de l'instrument médical et/ou de la chambre de sorte que la température de l'instrument médical soit inférieure à la température de la chambre de façon à permettre à l'agent stérilisant de se condenser au moins partiellement sur l'instrument.

**14.** Procédé de stérilisation d'un instrument médical, comportant :

- le placement de l'instrument médical dans une chambre ;
- la fourniture d'un flux de gaz humidifié à l'instrument médical, dans lequel le flux de gaz humidifié présente une humidité spécifique contrôlée et est obtenu en :

- fournissant un flux de gaz dans un réservoir qui stocke un volume d'eau,
- contrôlant une température de l'eau et/ou une pression du gaz fourni pour l'humidité spécifique requise,
- faisant circuler le flux de gaz humidifié ayant l'humidité spécifique contrôlée depuis le réservoir.

**15.** Procédé selon la revendication 14, comportant le fait que le flux de gaz pénètre dans le réservoir sous la surface de l'eau, et/ou comportant la circulation du flux de gaz humidifié ayant l'humidité spécifique contrôlée depuis une tête de gaz à l'intérieur du réservoir.

**16.** Procédé selon la revendication 14 ou 15, comportant l'utilisation d'un processeur pour rechercher une valeur d'humidité spécifique souhaitée et pour déterminer des valeurs pour une température requise et/ou une pression requise sur la base de la valeur d'humidité spécifique souhaitée.

**17.** Procédé selon l'une quelconque des revendications 14 à 16, qui utilise un capteur de température, et un contrôleur de température destiné à maintenir l'eau à la température contrôlée.

**18.** Procédé selon les revendications 16 et 17, comportant le processeur qui reçoit la valeur de température contrôlée en guise d'entrée, et qui détermine la pression requise sur la base de l'humidité spécifique souhaitée et de la température contrôlée.

**19.** Procédé selon l'une quelconque des revendications 14 à 17, comportant le contrôle de la pression du gaz à l'aide d'un régulateur de pression destiné à maintenir la pression dans le réservoir à une pression contrôlée.

**20.** Procédé selon les revendications 16 et 19, comportant le processeur qui reçoit la valeur de pression contrôlée en guise d'entrée, et qui détermine la température requise sur la base de l'humidité spécifique souhaitée et de la pression contrôlée.

**21.** Procédé selon l'une quelconque des revendications 14 à 20, comportant (i) le processeur qui contrôle la température et/ou la pression dans le réservoir sur la base de la température requise et/ou de la pression requise, et/ou (ii) le processeur qui reçoit une indication représentative d'un paramètre de stérilisation souhaité et qui détermine la valeur d'humidité spécifique souhaitée sur la base de celui-ci, et/ou (iii) au moins l'ionisation partielle du flux de gaz humidifié ayant l'humidité spécifique contrôlée à l'aide d'une source de plasma ayant de fournir un flux de gaz humidifié à l'instrument médical.

**22.** Procédé selon la revendication 21, qui fait circuler le flux de gaz humidifié ayant l'humidité spécifique contrôlée entre le réservoir et la source plasma via un étrangleur.

**23.** Procédé selon l'une quelconque des revendications 14 à 22, comportant le maintien automatique du niveau d'eau dans le réservoir dans un intervalle prédéterminé.

**24.** Procédé selon la revendication 21 ou 22, comportant la mesure d'un ou plusieurs de l'humidité relative, de la température, de la pression et du débit entre le réservoir et la source de plasma.

**25.** Procédé selon l'une quelconque des revendications 14 à 24, comportant le contrôle d'une température de l'instrument médical et/ou de la chambre de sorte que la température de l'instrument médical soit inférieure à la température de la chambre afin de permettre à l'agent stérilisant de se condenser au moins partiellement sur l'instrument.

FIG. 1

FIG. 2

FIG. 3

200

202

Supply air stream to
plasma source

204

Ionize air

206

Block chamber for
sterilizing mixture

208

Reduce pressure in
chamber

210

Supply air stream to
chamber via choke

212

Supply air stream to
instruments

214

Sterilizing agent
(partially) condenses
on instrument

216

Open bypass

218

Take instrument
from chamber

FIG. 4

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110027125 A1

- EP 3228550 A

**Non-patent literature cited in the description**

- **S. MOREAU et al.** Using the flowing afterglow of a plasma to inactivate Bacillus subtilis spores: Influence of the operating conditions. *J. Appl. Phys.*, 15 July 2000, vol. 88 (2)